# EUROPEAN PATENT SPECIFICATION

(11) **EP 1 757 683 B1**
(45) Date of publication and mention of the grant of the patent: **28.03.2012**
(21) Application number: 06076525.2
(22) Date of filing: 01.06.2001
(51) Int. Cl.: C12N 9/22, C12P 21/02, C12Q 1/68

(54) **Method of making a mutated TypeIIT endonuclease which has nicking activity**
Verfahren zur Herstellung von einer mutierten TypIIT Endonuklease die nicking Aktivität aufweist.
Procede de manufacture d'une endonuclease Type IIT mutée a activité de coupre

(30) Priority: 02.06.2000 US 586935
(43) Date of publication of application: 28.02.2007
(62) Divisional of application: 01939836.1
(73) Proprietor: NEW ENGLAND BIOLABS, INC., Ipswich, MA 01938-2723 (US)
(72) Inventor: Kong, Huimin, Wenham, MA 01984 (US); Higgins, Lauren, Rockport, MA 01966 (US); Dalton, Michael, Manchester, MA 01944 (US); Kucera, Rebecca, Hamilton, MA 01982 (US); Schildkraut, Ira, Cerrillos, NM 87010 (US); Wilson, Geoffrey, South Hamilton, MA 01982 (US)
(74) Representative: Froud, Clive

(56) References cited:
- EP-A- 0 497 272
- DATABASE BIOSIS [Online] BIOSCIENCES INFORMATION SERVICE, PHILADELPHIA, PA, US; 1996, ABDURASHITOV M A ET AL: "N. BstSe, a site-specific nicase from Bacillus stearothermophilus SE-589" XP002274443 Database accession no. PREV199799813588 & MOLEKULYARNAYA BIOLOGIYA (MOSCOW), vol. 30, no. 6, 1996, pages 1261-1267, ISSN: 0026-8984
- MORGAN RICHARD D ET AL: "Characterization of the specific DNA nicking activity of restriction endonuclease N.BstNBI" BIOLOGICAL CHEMISTRY, vol. 381, no. 11, November 2000 (2000-11), pages 1123-1125, XP001009735 ISSN: 1431-6730
- HIGGINS LAUREN S ET AL: "The nicking endonuclease N.BstNBI is closely related to Type IIs restriction endonucleases Mlyl and Plel" NUCLEIC ACIDS RESEARCH, OXFORD UNIVERSITY PRESS, SURREY, GB, vol. 29, no. 12, 15 June 2001 (2001-06-15), pages 2492-2501, XP002212150 ISSN: 0305-1048
- BESNIER C E ET AL: "Converting MlyI endonuclease into a nicking enzyme by changing its oligomerization state." EMBO REPORTS. SEP 2001, vol. 2, no. 9, September 2001 (2001-09), pages 782-786, XP002212151 ISSN: 1469-221X

## Description

### BACKGROUND OF THE INVENTION

In accordance with this invention, procedures to identify and create site-specific nicking enzymes are described, and suitability of their application to SDA in the absence of modified nucleotides such as α-thio nucleotides is demonstrated.

This application is a Divisional of EP-A-01939836.1 (EP 1303530-B).

The use of nicking endonucleases in strand-displacement amplification application (SDA) liberates such amplification from the technical limitation of employing modified (particularly α-thiophosphate substituted) nucleotides.

Restriction endonucleases are enzymes that recognize and cleave specific DNA sequences. Usually there is a corresponding DNA methyltransferase that methylates and therefore protects the endogenous host DNA from the digestion of a certain restriction endonuclease. Restriction endonucleases can be classified into three groups: type I, II, and III. More than 3000 restriction endonucleases with over two hundred different specificities have been isolated from bacteria (Roberts and Macelis, Nucleic Acids Res. 26:338-350 (1998)). Type II and type IIs restriction enzymes cleave DNA at a specific position, and therefore are useful in genetic engineering and molecular cloning.

Most restriction endonucleases catalyze double-stranded cleavage of DNA substrates via hydrolysis of two phosphodiester bonds on two DNA strands (Heitman, Genetic Engineering 15:57-107 (1993)). For example, type II enzymes, such as EcoRI and EcoRV, recognize palindromic sequences and cleave both strands symmetrically within the recognition sequence. Type IIs endonucleases recognize asymmetric DNA sequences and cleave both DNA strands outside of the recognition sequence.

There are some proteins in the literature which break only one DNA strand and therefore introduce a nick into the DNA molecule. Most of those proteins are involved in DNA replication, DNA repair, and other DNA-related metabolisms (Kornberg and Baker, DNA replication. 2nd edit. W.H. Freeman and Company, New York, (1992)). For example, gpII protein of bacteriophage fI recognizes and binds a very complicated sequence at the replication origin. It introduces a nick in the plus strand, which initiates rolling circle replication, and it is also involved in circularizing the plus strand to generate single-stranded circular phage DNA. (Geider et al., J. Biol. Chem. 257:6488-6493 (1982); Higashitani et al., J. Mol. Biol. 237:388-400 (1994)). Another example is the MutH protein, which is involved in DNA mismatch repair in *E. coli*. MutH binds at dam methylation sites (GATC), where it forms a protein complex with nearby MutS which binds to a mismatch. The MutL protein facilitates this interaction and this triggers single-stranded cleavage by MutH at the 5' end of the unmethylated GATC site. The nick is then translated by an exonuclease to remove the mismatched nucleotide (Modrich, J. Biol. Chem. 264:6597-6600 (1989)).

The nicking enzymes mentioned above are not very useful in the laboratory for manipulating DNA due to the fact that they usually recognize long, complicated sequences and usually associate with other proteins to form protein complexes which are difficult to manufacture. Thus none of these nicking proteins are commercially available. Recently, we have found a nicking protein, N.*Bst*NBI, from the thermophilic bacterium *Bacillus stearothermophilus*, which is an isoschizomer of N.*Bst*SEI (Abdurashitov et al., Mol. Biol. (Mosk) 30:1261-1267 (1996)). Unlike gpII and MutH, N.*Bst*NBI behaves like a restriction endonuclease. It recognizes a simple asymmetric sequence, 5' GAGTC 3', and it cleaves only one DNA strand, 4 bases away from the 3'-end of its recognition site (Fig. 1A).

Because N.*Bst*NBI acts more like a restriction endonuclease, it should be useful in DNA engineering. For example, it can be used to generate a DNA substrate containing a nick at a specific position. N.*Bst*NBI can also be used to generate DNA with gaps, long overhangs, or other structures. DNA templates containing a nick or gap are useful substrates for researchers in studying DNA replication, DNA repair and other DNA related subjects (Kornberg and Baker, DNA replication. 2nd edit. W.H. Freeman and Company, New York, (1992)). A potential application of the nicking endonuclease is its use in strand displacement amplification (SDA), which is an isothermal DNA amplification technology. SDA provides an alternative to polymerase chain reaction (PCR), and it can reach 10⁶-fold amplification in 30 minutes without thermo-cycling (Walker et al., Proc. Natl. Acad. Sci. USA 89:392-396 (1992)). SDA uses a restriction enzyme to nick the DNA and a DNA polymerase to extend the 3'-OH end of the nick and displace the downstream DNA strand (Walker et al., (1992)). The SDA assay provides a simple (no temperature cycling, only incubation at 60°C) and very rapid (as short as 15 minutes) detection method and can be used to detect viral or bacterial DNA. SDA is being introduced as a diagnostic method to detect infectious agents, such as *Mycobacterium tuberculosis* and *Chlamydia trachomatis* (Walker and Linn, Clin. Chem. 42:1604-1608 (1996); Spears et al., Anal. Biochem. 247:130-137 (1997)).

For SDA to work, a nick has to be introduced into the DNA template by a restriction enzyme. Most restriction endonucleases make double-stranded cleavages. Therefore, modified α-thio deoxynucleotides (dNTPαS) have to be incorporated into the DNA, so that the endonuclease only cleaves the unmodified strand which is within the primer region (Walker et al., 1992). The α-thio deoxynucleotides are eight times more expensive than regular dNTPs (Pharmacia), and are not incorporated well by the *Bst* DNA polymerase as compared to regular deoxynucleotides (J. Aliotta, L. Higgins, and H. Kong, unpublished observation).

Alternatively, it has been found that if a nicking endonuclease is used in SDA, it will introduce a nick into the DNA template naturally. Thus the dNTPαS is no longer needed for the SDA reaction when a nicking endonuclease is being used. This idea has been tested, and the result agreed with our speculation. The target DNA can, for example, be amplified in the presence of the nicking endonuclease N.*Bst*NBI, dNTPs, and *Bst* DNA polymerase. Other nicking endonucleases can also be used. It is even possible to employ a restriction endonuclease in which the two strands are cleaved sequentially, such that nicked intermediates accumulate.

With the advent of genetic engineering technology, it is now possible to clone genes and to produce the proteins that they encode in greater quantities than are obtainable by conventional purification techniques. Type II restriction-modification systems are being cloned with increasing frequency. The first cloned systems used bacteriophage infection as a means of identifying or selecting restriction endonuclease clones (*Eco*RII: Kosykh et al., Molec. Gen. Genet 178:717-719 (1980); *Hha*II: Mann et al., Gene 3:97-112 (1978); *Pst*I: Walder et al., Proc. Nat. Acad. Sci. 78:1503-1507 (1981)). Since the presence of restriction-modification systems in bacteria enable them to resist infection by bacteriophages, cells that carry cloned restriction-modification genes can, in principle, be selectively isolated as survivors from libraries that have been exposed to phage. This method has been found, however, to have only limited value. Specifically, it has been found that cloned restriction-modification genes do not always manifest sufficient phage resistance to confer selective survival.

Another cloning approach involves transferring systems initially characterized as plasmid-borne into *E.* coli cloning plasmids (*Eco*RV: Bougueleret et al., Nucl. Acids Res. 12:3659-3676 (1984); PaeR7: Gingeras and Brooks, Proc. Natl. Acad. Sci. USA 80:402-406 (1983); Theriault and Roy, Gene 19:355-359 (1982); PvuII: Blumenthal et al., J. Bacteriol. 164:501-509 (1985)).

A further approach which is being used to clone a growing number of systems involves selection for an active methylase gene (refer to U.S. Patent No. 5,200,333 and BsuRI: Kiss et al., Nucl. Acids Res. 13:6403-6421 (1985)). Since restriction and modification genes are often closely linked, both genes can often be cloned simultaneously. This selection does not always yield a complete restriction system however, but instead yields only the methylase gene (*Bsp*RI: Szomolanyi et al., Gene 10:219-225 (1980); BcnI: Janulaitis et al, Gene 20:197-204 (1982); BsuRI: Kiss and Baldauf, Gene 21:111-119 (1983); and *Msp*I: Walder et al., J. Biol. Chem. 258:1235-1241 (1983)).

Another method for cloning methylase and endonuclease genes is based on a colorimetric assay for DNA damage (see U.S. Patent No. 5,492,823). When screening for a methylase, the plasmid library is transformed into the host E. coli strain such as AP1-200. The expression of a methylase will induce the SOS response in an *E. coli* strain which is McrA+, McrBC+, or Mrr+. The AP1-200 strain is temperature sensitive for the Mcr and Mrr systems and includes a lac-Z gene fused to the damage inducible *din*D locus of *E. coli*. The detection of recombinant plasmids encoding a methylase or endonuclease gene is based on induction at the restrictive temperature of the *lac*Z gene. Transformants encoding methylase genes are detected on LB agar plates containing X-gal as blue colonies. (Piekarowicz et al., Nucleic Acids Res. 19:1831-1835 (1991) and Piekarowicz et al., J. Bacteriology 173:150-155 (1991)). Likewise, the E. *coli* strain ER1992 contains a *din*D1-*Lac*Z fusion but is lacking the methylation dependent restriction systems McrA, McrBC and Mrr. In this system (called the "endo-blue° method), the endonuclease gene can be detected in the absence of its cognate methylase when the endonuclease damages the host cell DNA, inducing the SOS response. The SOS-induced cells form deep blue colonies on LB agar plates supplemented with X-gal. (Fomenkov et al., Nucleic Acids Res. 22:2399-2403 (1994)).

Sometimes the straight-forward methylase selection method fails to yield a methylase (and/or endonuclease) clone due to various obstacles (see, e.g., Lunnen et al., Gene 74(1):25-32 (1988)). One potential obstacle to cloning restriction-modification genes lies in trying to introduce the endonuclease gene into a host not already protected by modification. If the methylase gene and endonuclease gene are introduced together as a single clone, the methylase must protectively modify the host DNA before the endonuclease has the opportunity to cleave it. On occasion, therefore, it might only be possible to clone the genes sequentially, methylase first then endonuclease (see U.S. Patent No. 5,320,957).

Another obstacle to cloning restriction-modification systems lies in the discovery that some strains of *E. coli* react adversely to cytosine or adenine modification; they possess systems that destroy DNA containing methylated cytosine (Raleigh and Wilson, Prog. Natl. Acad. Sci. USA 83:9070-9074 (1986)) or methylated adenine (Heitman and Model, J. Bacteriology 196:3243-3250 (1987); Raleigh et al., Genetics 122:279-296 (1989); Waite-Rees et al., J. Bacteriology 173:5207-5219 (1991)). Cytosine-specific or adenine-specific methylase genes cannot be cloned easily into these strains, either on their own, or together with their corresponding endonuclease genes. To avoid this problem it is necessary to use mutant strains of *E. coli* (McrA-and McrB- and Mrr-) in which these systems are defective.

An additional potential difficulty is that some restriction endonuclease and methylase genes may not express in *E. coli* due to differences in the transcription machinery of the source organism and *E. coli*, such as differences in promoter and ribosome binding sites. The methylase selection technique requires that the methylase express well enough in E. coli to fully protect at least some of the plasmids carrying the gene.

Because purified restriction endonucleases, and to a lesser extent modification methylases, are useful tools for characterizing genes in the laboratory, there is a commercial incentive to obtain bacterial strains through recombinant DNA techniques that synthesize these enzymes in abundance. Such strains would be useful because they would simplify the task of purification as well as provide the means for production in commercially useful amounts.

### SUMMARY OF THE INVENTION

The invention provides a method of making a mutated Type IIT endonuclease which has nicking activity

The method of the invention is defined in the attached claims.

The present specification describes a method of using nicking endonuclease such as N.*Bst*NBI in the absence of modified nucleotides such as α-thio dNTPs in strand displacement amplification is disclosed.

Additional examples of non-modified strand displacement amplification mediated by four additional enzymes generated by engineering of other nucleases is also disclosed. An example of non-modified strand displacement amplification mediated by a restriction endonuclease with a nicked intermediate is disclosed-Finally, approaches for constructing such nicking endonucleases are disclosed.

### BRIEF DESCRIPTION OF THE DRAWINGS

(Figures 1 - 6 deleted)

Figure 7 shows the result of non-modified strand displacement amplification using nicking enzyme N.*Bst*NHI. Lane 1 shows the molecular weight standards and Lane. 2 shows the 160-bp DNA fragment produced from SDA by N.*Bst*NBI, which is vindicated by the arrow head.

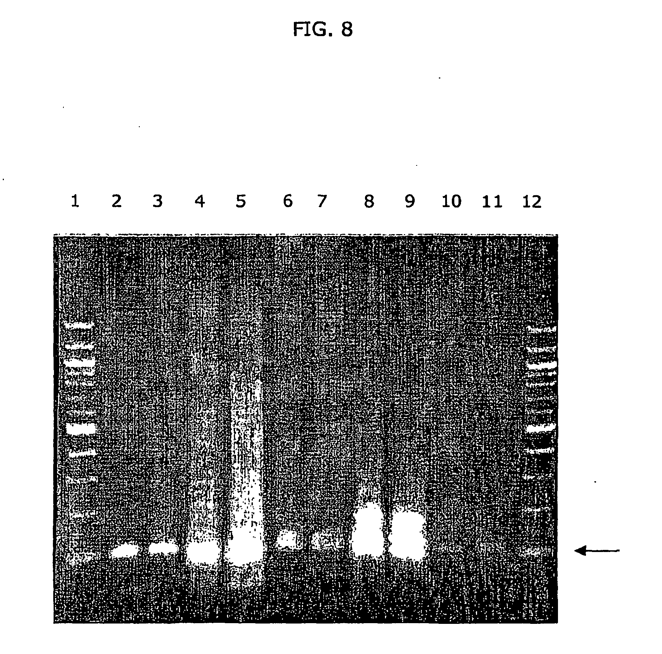

Figure 8 shows the result of non-modified strand displacement amplification using five nicking enzymes, with duplicate samples nun. Lanes 1 and 12 are the molecular weight marker lanes (100 bp ladder). Lanes 2 and 3, N.BstNBI; lanes 4 and 5, N.AlwI; lanes 6 and 7 N.MlyI; lanes 8 and 9, N.BbvCI-1-35; lanes 10 and 11, BbvCI-2-12. Arrow indicates the position of the expected 100-120 bp product bands.

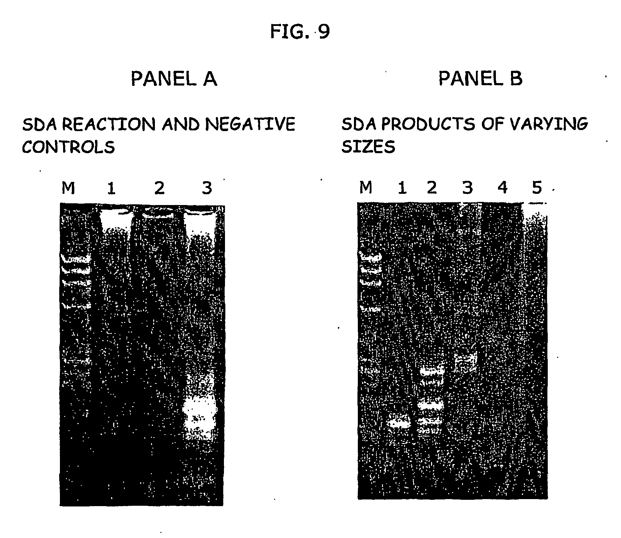

Figure 9 shows the result of non-modified strand displacement amplification using BsrFI, an enzyme that cleaves in two steps. Panel A, SDA reactions as described in Example 6 with: lane 1, no DNA substrate, no product appearing; lane 2, no BsrFI, no product appearing; lane 3, complete reaction, 150 bp amplicon appearing. M= size standard markers *Hae*III digest of φX174; Panel B, SDA reactions as described in Example 6 but with different DNA substrates leading to different sized amplicons: Lane 1, 150 bp product; lane 2 - 190 bp product; lane 3 - 330 bp product; lane 4 - 430 bp product; lane 5 - 500 bp product. M= size standard markers HaeIII digest of φX174

### DETAILED DESCRIPTION OF THE INVENTION

In accordance this invention, procedures to create site-specific nicking enzymes are described. The suitability of their application to SDA in the absence of modified nucleotides such as α-thio nucleotides is demonstrated.

Those skilled in the art will appreciate that for use in SDA, a nicking enzyme must have sequence-specificity in that activity, so that a single nick can be introduced at the location of the desired priming site. In SDA as conventionally applied, the sequence-specific nicking activity derives from two factors: the sequence-specificity of the restriction endonuclease employed and the strand-specificity enforced by the employment of modified (e.g. α-thiophosphate substituted, boron-substituted (α-boronated) dNTPs or cytosine-5 dNTP) nucleotides. This procedure increases the cost (due to the expense of the modified nucleotides) and reduces the length of the amplicon that can be synthesized (due to poor incorporation by the polymerase).

Both sequence specificity and strand specificity can be obtained in an enzyme as found in the original host, exemplified by N.BstNBI.

The cloning of the N.*Bst*NBI restriction endonuclease gene from *Bacillus stearothermophilus* 33M (NEB #928, New England Biolabs, Inc., Beverly, MA) proved to be challenging. A methylase selection strategy was tried and one methylase expression clone was isolated. However, the flanking ORFs did not encode the N.*Bst*NBI nicking enzyme. This turned out to be an orphan methylase, i.e., a methylase not associated with the cognate endonuclease gene. The method by which the N.*Bst*NBI nicking endonuclease was preferably cloned and expressed in E. coli is described herein:
1. Purification of the N.*Bst*NBI restriction endonuclease to near homogeneity and N-terminal and internal amino acid sequence determination.
   Nine chromatography columns were used to purify the N.*Bst*NBI endonuclease protein. They included an XK 50/14 fast flow P-cell column, an HR 16/10 Source^{™} 15Q, five HR 16/10 Heparin-TSK-Guardgel columns, an HR 10/10 Source^{™} 15Q column and a Resource^{™} 15S. The purification yielded one protein band at approximately 72 kDa on an SDS-PAGE protein gel following Coomassie blue staining. The N-terminal 31 amino acid residues were determined by sequential degradation of the purified protein on an automated sequencer. To determine its internal protein sequence, a 6-kDa polypeptide fragment was obtained following cyanogen bromide digestion of the 72-kDa N.*Bst*NBI protein. The first 13 amino acid residues of this 6-kDa were determined. This 13-amino acid sequence differs from the sequence of the N-terminal 31 amino acid residues, suggesting it was internal N.*Bst*NBI protein sequence.
2. Amplification of a segment of the N.*Bst*NBI endonuclease gene and subsequent cloning.
   Degenerate primers were designed based on both the N-terminal and internal amino acid sequences. These primers were used to PCR amplify the 5' end of the endonuclease gene. PCR products were cloned into plasmid pCAB16 and sequenced. The approximately 1.4 kb PCR fragment was then identified by comparing the amino acid sequences deduced from the cloned DNA with the N-terminal amino acid sequence of the N.*Bst*NBI endonuclease protein.
3. Chromosome walking via inverse PCR to isolate the N.*BstNBI* endonuclease and methylase gene.
   To clone the entire N.*Bst*NBI endonuclease gene as well as its corresponding DNA methylase gene, inverse PCR techniques were adopted to amplify DNA adjacent to the original 1.4 kb endonuclease gene fragment (Ochman et al., Genetics 120:621 (1988); Triglia et al., Nucl. Acids Res. 16:8186 (1988) and Silver and Keerikatte, J. Cell. Biochem. (Suppl.) 13E:306, Abstract No. WH239 (1989)). In total, two rounds of inverse PCR were performed. At that point, the endonuclease and the methylase open reading frames (ORF) were identified (Figure 1B)
   The endonuclease gene (*n.bstNBIR*) turned out to be a 1815-bp ORF that codes for a 604-amino acid protein with a deduced molecular weight of 70,368 Daltons (Figure 2). This agreed with the observed molecular mass of the N.*Bst*NBI endonuclease that was purified from native *Bacillus Stearothermophilus* 33M. Close to the endonuclease gene a 906-bp ORF, *n.bstNBIM*, was found. It was oriented in a convergent manner relative to the endonuclease (Figure 1B). The protein sequence deduced from the *n.bstNBIM* gene shares significant sequence similarity with other adenine methylases (Figure 3).
4. Expression of N.*Bst*NBI endonuclease gene using pHKUV5 and pHKT7 plasmids.
   The two-step method for cloning restriction-modification systems is described in U.S. Patent No. 5,320,957. The first step is protection of the host cell from corresponding endonuclease digestion by pre-modification of recognition sequences. This is accomplished by introducing the methylase gene into a host cell and expressing the gene therein. The second step includes introduction of the endonuclease gene into the pre-modified host cell and subsequent endonuclease production.

The *pleIM* gene (Figure 4) was cloned into plasmid pHKUV5 (Figure 5) and transformed into *E. coli* cells. As a result, the *E. coli* cells were modified by the pHKUV5-*pleIM*. In this case, the PleI methylase (*pleIM*) was used for pre-modification of the host cells because *Ple*I and N.*Bst*NBI share the same recognition sequence.

The endonuclease gene, *n.bstNBIR*, was cloned into pHKT7 (Figure 6), and then introduced into *E. coli* ER2566 containing pHKUVB-pleIM. The culture was grown to middle log and then induced by the addition of IPTG to a final concentration of 0.4 mM. The yield of recombinant N.*Bst*NBI endonuclease is 4 X 10⁷ units per gram cells.

Appropriate cleavage specificity for SDA can be enabled by mutational alteration of enzymes having double-stranded cleavage activity. In one approach :, the sequence specificity is conferred by the specificity of a restriction enzyme, as in conventional SDA, but the strand specificity is engineered into it by mutation, so that a single purified enzyme recognizes a specific sequence and specifically nicks only one strand. Three distinct approaches to obtaining strand-specificity (nicking activity) have been devised and exemplified. Each enables performance of SDA in the absence of α-thio nucleotides. These approaches are described hereinbelow.

### 1. Identification of suitable target enzymes for engineering into nicking enzymes

Sequence-specific restriction endonucleases can be identified by methods well known in the art, and many approaches to cloning these have been devised, as described above. For the present invention, two subclasses of restriction endonucleases can be identified that are preferred starting materials for creation of sequence-specific nicking endonucleases. These will be referred to below as subclass A and subclass B. For one of these classes, the approach to obtaining mutants that nick specifically is divided into two subsets, to be referred to as subclass A1 and subclass A2. Isolation and characterization of mutants as described in subclass A

will be summarized here. Isolation and characterization of mutants of subclass B enzymes will be described in detail here.

Both classes of enzymes are found among those listed in REBASE (http://rebase.neb.com/rebase.charts. htm1 "Type Iis enzymes" link; Roberts and Marcelis, Nucleic Acids Res. 29:368-269 (2001)) as Type IIS endonucleases. These can be identified among restriction endonucleases as those in which the recognition site is asymmetric.

However, specifically those enzymes belonging to subclass A are frequently referred to as 'Type IIS' endonucleases (Szybalski, Gene 100:13-26 (1991)). These enzymes recognize asymmetric sequences and cleave the DNA outside of, and to one side of, the recognition sequence. The examples that have been studied each comprise an N-terminal sequence-specific DNA binding moiety, joined with a C-terminal sequence-non-specific cleavage moiety by zero or more amino acids.

Enzymes belonging to subclass B are often referred to as 'Type IIT' endonucleases (Kessler, et al., Gene 47:1-153 (1986); Stankevicius, et al. Nucleic Acids Res. 26:1084-1091 (1998)), or alternately as 'Type IIQ' endonucleases (Degtyarev, et al., Nucleic Acids Res. 18:5807-5810 (1990); Degtyarev, et al., Nucleic Acids Res. 28:e56 (2000)). These enzymes also recognize asymmetric sequences but they cleave the DNA within the recognition sequence.

Methods for identifying and characterizing the recognition site of a restriction endonuclease are well-known in the art. In addition, a list of the known enzymes belonging to these, and other, groups may be obtained from REBASE at http://rebase.neb.com.

### 2. Creation of nicking mutants from subclass A (Not According to the Invention)

The subclass A enzymes studied were *Fok*I, *Mly*I, PleI, and *Alw*I. Enzymes of this subclass are thought to act symmetrically with respect to strand-cleavage. The C-terminal domains of two identical protein molecules are believed to interact transiently during DNA cleavage to form a homodimer.

Two of the enzymes disclosed in the present specification were derived from subclass A enzymes in one of two ways. In one method . (method A1) cleavage of one of the two DNA strands was suppressed by mutating, within the endonuclease gene, the region coding for the dimerization interface that is needed for double-strand cleavage, such that only one cleavage occurs. This mutation may comprise alteration of particular residues required for dimerization individually or together.

In the other method . (method A2), cleavage of one of the two strands was suppressed by substitution of the region of the endonuclease containing the dimerization interface with a corresponding region from an endonuclease known to be dimerization-defective. This region may be obtained from a portion of a gene such as the gene encoding N.*Bst*NBI, the endonuclease of the present invention described above, or may be obtained from other naturally-occurring or from engineered genes containing this dimerization function.

### 3. Creation of nicking mutants from subclass B. (The Method of the Invention)

The fourth and fifth nicking endonucleases disclosed herein were derived from the enzyme BbvCI, a member of subclass B. Enzymes of subclass B are thought to act symmetrically with respect to strand-cleavage. They are envisaged to be functionally heterodimeric, that is to say to comprise two different subunits, or domains, each with its own catalytic site. In the active enzyme, the two subunits, or domains, interact to achieve DNA recognition together, and to catalyze double-strand cleavage. Of four subclass B enzymes studied-*Aci*I, *Bsr*BI, *Bss*SI, and *Bbv*CI-only *Bbv*CI comprised two different protein subunits. The other three enzymes were single proteins each of which, we presume, comprises two different domains. In principle, nicking mutants can be made from either kind of enzyme, although doing so is more straightforward using enzymes that, like BbvCI, comprise separate, rather than joined, subunits.

### A. Identification of heterodimeric enzymes of subclass B.

Heterodimeric members of the subclass may be recognized in two ways: by analysis of endonuclease purified from the original organism or from a recombinant host containing the cloned restriction system, or by sequence analysis of the cloned restriction system. In the former case, the purified endonuclease may be characterized by electrophoresis on SDS-PAGE, which will usually reveal the presence of two protein components migrating at different positions. It may be the case that the two subunits, although distinct in sequence and the products of different genes, still migrate at the same mobility on SDS-PAGE. This situation will be recognized, cause the apparent molecular weight derived from SDS-PAGE analysis will be one-half of the apparent molecular weight derived from gel-filtration analysis. Further, the N-terminal amino acid sequence analysis of the purified endonuclease will reveal the presence of two different amino acids at each sequencing cycle, in the apparently single band. Procedures for determining these properties are well known in the art, and are disclosed for example in Current Protocols in Protein Analysis (sections 8.3, 10.1, and 11.10; Coligan, F.E., Dunn, B.M., Ploegh, H.L., Speicher, D.W., and Wingfield, P.T. Current Protocols in Protein Science, John Wiley and Sons, (1997)).

In the latter analysis, the restriction systems amenable to this invention will contain up to four open reading frames, two encoding methyltransferases (one for each strand of the asymmetric site), and two encoding the subunits of the restriction endonuclease. The open reading frames encoding the methyltransferases may be recognized by sequence analysis according to Malone, et al., J. Mol. Biol. 253:618-632 (1995)). Additional open reading frames may also be present including those involved in the regulation of gene expression (such as C proteins), and in the repair of damage resulting from the deamination of methylated cytosine (such as Vsr proteins).

### B. Verification of the heterodimeric character of enzymes identified by sequence analysis.

Genes encoding subunits of the endonuclease may be verified by creating expression clones in which the methyltransferase genes are carried on one plasmid, and the candidate endonuclease genes are carried on one or more additional plasmid(s), as disclosed in Brooks, et al. (US Patent 5,320,957). Expression hosts carrying only the methyltransferase plasmid(s) will cause DNA within the cell to be resistant to action of the endonuclease, but will express no endonuclease activity. Addition of the endonuclease genes on the additional plasmid(s) will result in expression of the endonuclease activity in crude extracts of the recombinant host. In some situations it may be possible to express the endonuclease genes in the absence of the methyltransferase genes, as disclosed in WO 99/11821.

The requirement for both open reading frames for endonuclease activity may be verified by (i) creation of expression clones in which each of the two open reading frames can be expressed separately, e.g. by placing each open reading frame on a separate compatible plasmid, or by placing each open reading frame under the control of a promoter that can be induced separately (e.g. inducible by lactose or by arabinose) and then testing for expression of the endonuclease when only one open reading frame is present or only one open reading frame is expressed. Endonuclease activity will be obtained only when both open reading frames are expressed. It may also be possible to reconstitute activity by mixing extracts from two recombinant hosts expressing each open reading frame separately. The requirement for both open reading frames may alternatively be verified by (ii) creation of deletion or insertion mutations in each of the candidate open reading frames separately, followed by assessment of endonuclease activity of the resulting recombinant host. For enzymes of subclass B, both wild-type open reading frames will be required for expression of the endonuclease.

### C. Converting a heterodimeric subclass B enzyme to a nicking enzyme.

Once an appropriate subclass B endonuclease has been identified, nicking enzyme derivatives pertinent to the present invention are obtained by inactivating the active site for cleavage in either subunit without interfering with the proper subsequent assembly of the enzyme. Appropriate mutations in the enzyme can be created by making mutational changes in amino acids, individually or in combination, that comprise the active site, or that influence its chemistry or organization; and then assessing the nicking activity of enzyme produced by each mutant. The magnitude of this effort may be reduced by focusing on regions conserved in several different but related enzymes.

Changes can be introduced by the steps of:
1. Identifying a conserved region by alignment of several members of this class of enzymes. Conceptual translations of five genes were employed: the two subunits of *Bbv*CI, termed *Bbv*CI-1, *Bbv*CI-2, and three conventional homodimeric type II endonucleases that recognize related, palindromic, sites: Bsu36I, BlpI, and DdeI. These genes exhibit limited homology in discrete, conserved, blocks. One conserved block contained the sequence EXK. This motif was judged to be the likely active site for cleavage, in which changes may be expected to abolish cleavage but still enable assembly of a conformationally native complex in which the other subunit would still be able to cleave. These were judged favorable sites for analysis.
2. Generating mutations within the favorable region by cassette mutagenesis. This process comprised the steps of:
   a) designing two mutagenic primers for inverse PCR, one for each gene, *bbvCI-1* and *bbvcI-2*. These mutagenic primers were designed such that the nucleotides encoding the EXK motive included 20% random nucleotides, and 80% the correct nucleotide at each of the nine positions. In each mutagenic primer, the region encoding the EXK motif was flanked by the unique sequence of the respective gene;
   b) conducting mutagenic PCR (as disclosed in Molecular Cloning, A Laboratory Manual, Sambrook, J. and Russel D.W., Cold Spring Harbor Laboratory, pp 8.81-8.95 (2001)) employing in separate reactions i) one mutagenic primer for *bbvCI-1* and a unique primer directed in the opposite direction from the mutagenic primer and immediately to its 5' side; and ii) one mutagenic primer for *bbvCI-2* and a unique primer directed in the opposite direction from the mutagenic primer and immediately to its 5' side, such that the entire plasmid vector was amplified;
   c) ligating the PCR products to form a population circular molecules;
   d) transforming an appropriate host (expressing both methyltransferases) separately with the two mutagenized populations targeting *bbvCI-1* and *bbvCI-2* to obtain colonies on selective plates; and
   e) for isolated members of each population, testing for cleavage activity in crude extracts, by the steps of
      i) growing cultures of the candidate colonies;
      ii) centrifuging the cultures to obtain cell pellets;
      iii) resuspending the cultures in lysis buffer;
      iv) lysing the resuspended cultures and clarifying them by centrifugation;
      v) withdrawing aliquots of the clarified extracts to assay tubes containing substrate plasmid DNA and digestion buffer;
      vi) incubating the assay tubes to allow enzyme-induced cleavage to occur; and
      vii) separating the plasmid DNA products by high-resolution gel electrophoresis and assessing whether no cleavage, single-strand cleavage, or double-strand cleavage, has occurred.

   Ideally, the substrate DNA is a plasmid that contains two or more well separated sites for cleavage. Under such circumstances, extracts containing inactive enzyme do not substantially alter the mobility of the various forms of the plasmid. Extracts containing wild-type enzyme abolish the supercoiled, linear and open-circular forms of the plasmid and produce two (or more) linear fragments in their place. And extracts containing nicking enzyme abolish the supercoiled plasmid form, converting it to open-circular form, without affecting the linear form.
3. Testing mutants that appear to nick by alternative procedures to confirm that they have this activity. Such procedures include, but are not limited to, sequencing through nicked sites and sequential nicking with complementary mutants, each defective in the activity of one of the two subunits.

Most preferably, candidate enzymes are tested by the first procedure, comprising the steps of:
a) incubating DNA containing at least one site for cleavage with purified or semi-purified enzyme;
b) purifying this DNA;
c) using it as a substrate for DNA sequencing across the site in both directions.

Nicking is indicated when the sequence in one direction continues across the site (i.e., the template strand is continuous) while the sequence in the other direction terminates abruptly at the site (i.e., the other strand is interrupted by a nick) .

In the second procedure, extracts of mutants thought to nick different strands are mixed together and the mixture is assayed for double-strand cleavage activity. While neither enzyme alone should catalyze double-strand cleavage, the mixture should be able to do so, either as a result of double-nicking, first on one strand by one enzyme, then on the complementary strand by the other, or by reassociation of the unmutated subunit of each enzyme to produce a fully-wild-type enzyme.

In this manner mutations in *Bbv*CI-1 and *Bbv*CI-2 were identified that enable cleavage of one strand but not the other at BbvCI sites. These are designated *Bbv*CI-1-37 and *Bbv*CI-2-12. The use of these enzymes in non-modified SDA is exemplified below.

In another embodiment, appropriate cleavage specificity for SDA is enabled by the use of enzymes having double-stranded cleavage activity, but in which cleavage occurs in two sequential steps, such that a small amount of nicked intermediate is observed during the course of double-strand cleavage.

Such enzymes that accumulate a nicked intermediate can be identified by the steps of:
a) forming a double-stranded circular substrate molecule (typically a plasmid) with one or more sites for the endonuclease;
b) incubating this substrate with small amounts of the endonuclease or for short times, such that at most 20% of substrate molecules have suffered a double-strand cleavage event;
c) separating the DNA products by high-resolution gel electrophoresis; and
d) assessing whether no cleavage, single-strand cleavage, or double-strand cleavage has occurred.

If no cleavage has occurred, in a suitable electrophoresis system containing an intercalating agent such as ethidium bromide, the substrate molecule will migrate faster than a linear DNA of the same size; if single strand cleavage has occurred, the substrate molecule will migrate slightly slower than a linear DNA of the same size; if a single double strand cleavage has occurred, the substrate molecule will migrate at the same position as a linear DNA of that size.

The nicked intermediates formed by such enzymes can support SDA as exemplified in Example 6.

Examples 1-3 deleted.

### EXAMPLE 4

### Non-modified Strand displacement amplification using N.BstNBI

### (Not according to the Invention)

For strand displacement amplification (SDA) to work, a nick has to be introduced into the DNA templates by a restriction enzyme.

Most restriction endonucleases make double stranded breaks and therefore, α-thio dNTPs have to be used in SDA. We have tested the nicking endonuclease N.*Bst*NBI in non-thio SDA and we found the target DNA could be successfully amplified. The following is the detailed protocol for non-thio SDA with N.*Bst*NBI.
1. Prepare mix A (below) in a plastic 1.5 ml tube at 4°C:

| Reagent Stock | Final Concentration | 40µl Volume |
|---|---|---|
| 250 mM KP04, (pH 7.5) | 35 mM KP04 | 7 µl |
| 2 M kCl | 100 mM | 2.5 µl |
| 4 mM each dNTP mix | 200 µM each dNTP | 2.5 µl |
| 100 mM DTT | 1 mM | 0.5 µl |
| 10 µM Primer 40 | 0.8 µM | 4 µl |
| 10 µM Primer 41 | 0.8 µM | 4 µl |
| 2.5 µM bump Primer 1 | 0.05 µM | 1 µl |
| 2.5 µM bump Primer 2 | 0.05 µM | 1 µl |
| 50 ng/µl DNA template | 1 ng/µl | 1 µl |
| H₂O | | 16.5 µl |

2. Denature at 100°C for 2 minutes; incubate at 55°C for 3 minutes to allow annealing of the primers. While these two temperature incubations are occurring, prepare mix B (below) in a separate plastic 1.5 ml tube and preincubate at 55°C for at least 30 seconds.

| Reagent Stock | Final Concentration | 10 µl Volume |
|---|---|---|
| 10X NEBuffer 2 | 1X | 5.0 µl |
| 10 mg/ml purified BSA | 100 µg/ml | 0.5 µl |
| 50 mM MgCl₂ | 2.5 mM MgC12 | 2.5 µl |
| 10 units/µl N.*Bst*NBI | 5 units per 50 µl | 0.5 µl |
| 20 nits/µl *Bst* DNA Pol 10 | units per 50 µl | 0.5 µl |
| H₂O | | 1 µl |

3. Add mix A to B; continue incubation at 55°C for 20-60 minutes, removing 10-20 µl volumes at different time points if desired; add to stop dye containing 0.2% SDS (final concentration).
4. Analyze by gel electrophoresis on high percentage agarose gels. Specific positive bands were observed on the agarose gel (Figure 7, Lane 1 = Molecular weight standard; Lane 2 = 160 bp band).
5. Description of primers (all flank the polylinker region of pUC19).
Primer 40: 5'-ACCGCATCGAATGCGAGTCGAGGACGACGGCCAGTG-3' (SEQ ID NO:24)
Primer 41: 5'-CGATTCCGCAATGCGAGTCGAGGCCATGATTACGCCAA-3' (SEQ ID NO:25)
Bump primer #1: 5'-CAGTCACGACGTT-3' (SEQ ID NO:26)
Bump primer #2: 5'-CACAGGAAACAGC-3' (SEQ ID NO:27)

6. Description of DNA template: The templates were constructed by cloning a short DNA duplex containing SphI site into pUC19 at EcoRI and HindIII sites to generate plasmid pUC19-SphI. Lambda DNA was digested by NlaIII and ligated into plasmid pUC19-SphI pre-digested with SphI. The DNA template, which was used to produce 160-bp DNA in SDA, was screened by PCR.

### EXAMPLE 5

### SDA Amplification with 5 Nicking Enzymes: N.BstNBI, N.MlyI, N.A1wI, BbvCI #2-12 and #1-35

### (Use of BbvCI mutants according to the Invention)

For strand displacement amplification (SDA) to work, a nick has to be introduced into the DNA template by a restriction enzyme.

Most restriction endonucleases make double stranded breaks and therefore, α-thio dNTPs have to be used in SDA. We have tested the nicking endonuclease N.BstNBI in non-modified SDA and we found the target DNA could be successfully amplified. The following is the detailed protocol for non-modified SDA with N.BstNBI. For N.MlyI, N.AlwI, BbvCI #2-12 and #1-35 non-modified SDA, modifications were made in the protocol in terms of the amount of enzyme used, the KCl and Mg concentrations, the assay temperature, the forward and reverse primers and the enzyme used to precut the plasmid template DNA. These modifications from the basic N.BstNBI non-modified SDA protocol are listed in part 4 of this Example.

### Non-modified SDA Protocol for N.BstNBI (with modifications for other enzymes listed)

1. Prepare mix A (below) in a plastic 1.5 ml tube at 4°C:

| Reagent Stock | Final Concentration | 35ul Volume |
|---|---|---|
| 250 mM tris, (pH 7.5) | 35 mM tris, (pH 7.5) | 7 ul |
| H20 | up to volume | 10.5 ul |
| 2 M KC1 | 100 mM | 2.5 ul |
| 4 mM each dNTP mix | 400 uM each DNTP | 5 ul |
| 10 mM DTT | 1 mM | 5 ul |
| 10 uM fw primer 33 | 0.2 uM | 1 ul |
| 10 uM rv primer 34 | 0.2 uM | 1 ul |
| 2.5 uM fw bump primer | 0.05 uM | 1 ul |
| 2.5 uM rv bump brimer | 0.05 uM | 1 ul |
| 50 ng/ul pre-cut pUCAH26* | 50 ng per 50 ul reaction | 1 ul |

2. Denature 100°C 2 minutes; incubate at 53°C for 3 minutes to allow annealing of the primers. While these two temperature incubations are occurring, prepare mix B (below) in a separate plastic 1.5 ml tube and preincubate at 55°C for 30 seconds.

| Reagent Stock | Final Concentration | 15 ul |
|---|---|---|
| H20 | up to volume | 3.5 ul |
| 1X NEBuffer 2 | 5 ul per 50 ul rxn vol | 5.0 ul |
| 10 mg/ml purified BSA | 100 ug/ml | 0.5 ul |
| 100 mM MgCl₂ | 10 mM MgC12 | 5.0 ul |
| 10 units/ul N.BstNB I | 5 units per 50 ul reaction | 0.5 ul |
| 20 units/ul Bst DNA Pol 10 | units per 50 ul reaction | 0.5 ul |

3. Add mix A to B; continue incubation at 53°C for 25 min. Add stop dye containing 0.2% SDS (final concentration) to 20 ul of the reaction volume.
4. Modifications in this protocol for other nicking enzymes; volumes of added water adjusted accordingly.

| Assay Component | | | | BbvCI | |
|---|---|---|---|---|---|
| | N.BstNB I | N.AlwI | N.MlyI | #1-35 | #2-12 |
| Amount of enzyme units | 5 | 10 | 10 | 10 | 5 |
| KCl concentration | 100 mM | 0 mM | 50 mM | 50 mM | 50 mM |
| MgC12 concentration | 10 mM | 10 mM | 5 mM | 10 mM | 5 mM |
| Temperature of assay | 53°C | 53°C | 53°C | 45°C | 45°C |
| Fw and Rv primer sets | P33,34 | P47,48 | P33,34 | P49,50 | P51,52 |
| Pre-cut plasmid templates (eliminates endogenous nick sites) | Precut by PleI | Precut by AlwI | Precut by PleI | Precut by PleI* | Precut by PleI* |

| | | | | | |
|---|---|---|---|---|---|
| *no endogenous BbvCI sites in pUC19; precutting not necessary | | | | | |

5. Analyze by gel electrophoresis on 1.5-1.8% agarose, or polyacrylamide gels. Specific 130-110 bp products were observed on the 1.8% agarose gel. (Figure 8)
6. Description of primers (all flank the polylinker region of pUC19).
Bump primers used with all 5 nicking enzymes:
   Bump forward primer:
      5'-CAGTCACGACGTT-3' (SEQ ID NO.:26)
   Bump reverse primer:
      5'-CACAGGAAACAGC-3' (SEQ ID NO:27)
Primers specific to the nicking enzymes:
   N.BstNB I and N.Mly I primers:
      P33forward:
         5'-ACCGCATCGAATGC**GAGTC**ATGTTACGACGGCCAGTG-3' (SEQ ID NO:28)
      P34reverse:
         5'-CGATTCCGCTCCAG**GAGTC**ACTTTCCATGATTACGCCAA-3' (SEQ ID NO:29)
   N.Alw I primers:
      P47forward:
         5'-ACCGCATCGAATGC**GGATC**ATGTTACGACGGCCAGTG-3' (SEQ ID NO:30)
      P48reverse:
         5'-CGATTCCGCTCCA**GGGATC**ACTTTCCATGATTACGCCAA-3' (SEQ ID NO:31)
   BbvC I, #1-35 primers:
      P49forward:
         5'-ACCGCATCGAATATGTATCGCCC**TCAGC**TACGACGGCCAGTG-3' (SEQ ID NO:32)
      P50reverse:
         5'-CGATTCCGCTCCAGACTTATCCC**TCAGC**TCCATGATTACGCCAA-3' (SEQ ID NO:33)
   BbvCI, #2-12 primers:
      P51forward:
         5'-ACCGCATCGAATATGTATCGC**GCTGAGG**TACGACGGCCAGTG-3' (SEQ ID NO:34)
      P52reverse:
         5'-CGATTCCGCTCCAGACTTATC**GCTGAGGT**CCATGATTACGCCAA-3 (SEQ ID NO:35)

7. Description of DNA template:
The templates were constructed by cloning a short DNA duplex containing a *Sph*I site into pUC19 at the EcoRI and *Hind*III sites to generate plasmid pUC19-SphI. DNA was digested by *Nla*III and ligated into plasmid pUC19-*Sph*I pre-digested with SphI. After selecting for different sized inserts into the vector backbone, a family of plasmids was selected that could be used in SDA protocols to generate different product lengths. The specific template used in this example, pUCAH26, generates a product length of 130-110 bp (product lengths before or after nick in SDA).

### EXAMPLE 6

### SDA Amplification With A Restriction Endonuclesae Possessing A Strong Nicking Intermediate, such as BsrFI

### (Not according to the Invention)

For strand displacement amplification (SDA) to work, a nick has to be introduced into the DNA template by a restriction enzyme. Most restriction endonucleases make double stranded breaks and therefore, modified nucleotides such as α-thio dNTPs have to be used in SDA. We have tested the nicking endonuclease N.BstNBI in non-modified SDA and we found the target DNA could be successfully amplified (Example 4). Another approach utilizes a restriction endonuclease possessing a strong nicking intermediate. Such enzymes, when provided with a supercoiled plasmid substrate, show an accumulation of a nicked circular DNA intermediate (one strand cut) before linearization of the DNA substrate (both strands cut). We tested a variety of thermostable restriction endonucleases for their ability to produce a nicking intermediate from a supercoiled plasmid substrate as a function of time, and developed an SDA protocol using one of these enzymes, BsrFI. The BsrFI restriction endonuclease accumulates a ten-fold higher level of nicked intermediate DNA products to linearized products as a function of time.

Non-thio SDA Protocol Utilizing a Restriction Enzyme Possessing a Strong Nicking Intermediate, BsrFI
1. Prepare mix A in a plastic Eppendorf tube:

| **Reagent Stock** | **Final Concentration** | **35ul Volume** |
|---|---|---|
| 250 mM KPO₄, (pH 7) | 35 mM KPO4 (pH 7) | 7 ul |
| H20 | up to volume | 18-13 ul |
| 500 mM KCl | 0-50 mM | 0-5 ul |
| 4 mM each dNTP mix | 400 uM each dNTP | 5 ul |
| 10 uM forward primer | 0.2 uM | 1 ul |
| 10 uM reverse primer | 0.2 uM | 1 ul |
| 2.5 uM bump primer | 0.05 uM | 1 ul |
| 2.5 uM bump primer | 0.05 uM | 1 ul |
| 50 ng/ul BsrFI precut DNA plasmid template | 50 ng per 50 ul reaction | 1 ul |

2. Denature 100°C 2 minutes; incubate at 55°C for 3 minutes to allow annealing of the primers. While these two temperature incubations are occurring, prepare mix B (below) in a separate plastic 1.5 ml tube and preincubate at 55°C for 30 seconds.

| **Reagent Stock** | **Final Concentration** | **15 ul** |
|---|---|---|
| H₂O | up to volume | 5.5ul |
| 1X NEBuffer 2 | 5 ul per 50 ul rxn vol | 5.0 ul |
| 10 mg/ml purified BSA | 100 ug/ml | 0.5 ul |
| 50 mM MgCl₂ | 2.5 mM MgCl₂. | 2.5 ul |
| 20 units/ul BsrF I | 10 units per 50 ul reaction | 0.5. ul |
| 10 units/ul Bst DNA Pol | 10 units per 50 ul reaction | 1.0 ul |

3. Add mix A to B; continue incubation at 55°C for 20-60 min. Add stop dye containing 0.2% SDS (final concentration) to 20 ul of the reaction volume to stop the reaction.
4. Analyze by gel electrophoresis on 1.5-1.8% agarose, or polyacrylamide gels. Specific 140-500 bp products were observed on the 1.8% agarose gel. (See section 7.)
5. Description of primers (all flank the polylinker region of pUC19).
Bump primers:
Bump forward primer:
   5'-CAGTCACGACGTT-3' (SEQ ID NO:26)
Bump reverse primer:
   5'-CACAGGAAACAGC-3' (SEQ ID NO:27)
Primers specific to BsrFI:
P13 forward:
   5'-ACCGCATCGAATGCATG**TACCGGC**TACGACGGCCAGTG-3' (SEQ ID NO:36)
P14 reverse:
   5'-CGATTCCGCTCCAGACTT**ACCGGC**TCCATGATTACGCCAA-3' (SEQ ID NO:37)

6. Description of DNA template:
The templates were a family of pUC19-modified plasmids. The endogenous single BsoBI and BamHI sites were eliminated by cut and subsequent fill-in reactions (elimination of the BamHI site was unrelated to this project), to form pRK22. Other related constructs were made by insertion of MspI-pBR322 fragments into AccI site of the pRK22 polylinker. This generated a family of related plasmids containing different lengths of inserts in the region of DNA amplified during SDA.

### SEQUENCE LISTING

> KONG. HUIMIN
   HIGGINS, LAUREN S.
   DALTON, MICHAEL
   KUCERA, REBECCA B.
   SCHILDKRAUT, IRA
   NEW ENGLAND BIOLABS, INC.
> Cloning And Producing The N.BstNBI Nicking Endonuclease And Related Methods For Using Nicking Endonucleases In Single-Stranded Displacement Amplification
> NEB-178-PCT
>
   >
> 09/586,935
   > 2000-06-02
>
> PatentIn Ver. 2.0
0> 24
   1> 36
   2> DNA
   3> Escherichia coli
0> 24
   gcatcga atgcgagtcg aggacgacgg ccagtg 36
0> 25
   1> 38
   2> DNA
   3> Escherichia coli
0> 25
   ttccgca atgcgagtcg aggccatgat tacgccaa 38
0> 26
   1> 13
   2> DNA
   3> Escherichia coli
0> 26
   tcacgac gtt 13
0> 27
   1> 13
   2> DNA
   3> Escherichia coli
0> 27
   aggaaac agc 13
0> 28
   1> 37
   2> DNA
   3> Unknown
0>
   3> Description of Unknown Organism:the last 13 bsed are from pUC19, the preceeding bases are random.
0> 28
   gcatcga atgcgagtca tgttacgacg gccagtg 37
0> 29
   1> 39
   2 > DNA
   3 > Unknown
0>
   3> Description of Unknown Organism: the last 15 bases are from pUC19, the preceeding bases are random.
0> 29
   ttccgct ccaggagtca ctttccatga ttacgccaa 39
0> 30
   1> 37
   2> DNA
   3> Unknown
0>
   3> Description of Unknown Organism:the last 13 bases rae from pUC19, the preceeding bases are random.
0> 30
   cgcatcga atgcggatca tgttacgacg gccagtg 37
0> 31
   1> 39
   2> DNA
   3> Unknown
0>
   3> Description of Unknown Organism:the last 15 bases are from pUC19, the preceeding bases are random.
0> 31
   ctccgct ccagggatca ctttccatga ttacgccaa 39
0> 32
   1> 42
   2> DNA
   3> Unknown
0>
   3> Description of Unknown Organism:the last 13 bases are from pUC19, the preceeding bases are random.
0> 32
   gcatcga atatgtatcg ccctcagcta cgacggccag tg 42
0> 33
   1> 44
   2> DNA
   3> Unknown
0>
   3> Description of Unknown Organism:the last 15 bases are from pUC19, the preceeding bases are random.
0> 33
   ttccgct ccagacttat ccctcagctc catgattacg ccaa 44
0> 34
   1> 42
   2> DNA
   3> Unknown
0>
   3> Description of Unknown Organism: the last 13 bases are from pUC19, the preceeding are random
> 34
   catcga atatgtatcg cgctgaggta cgacggccag tg 42
0> 35
   1> 44
   2> DNA
   3> Unknown
>
   0> Description of Unknown Organism:the last 15 bases are from pUC19, the preceeding bases are random.
3> 35
   ctccgct ccagacttat cgctgaggtc catgattacg ccaa 44
0> 36
   1> 38
   2> DNA
   3> Unknown
0>
   3> Description of Unknown Organism:the last 13 bases are from pUC19, the preceeding bases are random
0> 36
   catcga atgcatgtac cggctacgac ggccagtg 38
0> 37
   1> 40
   2> DNA
   3> Unknown
0>
   3> Description of Unknown Organism:the last 15 bases are from pUC19, the preceeding bases are random.
0> 37
   ctccgct ccagacttac cggctccatg attacgccaa 40

## Claims

1. A method according to claim 1 of making a mutated Type IIT endonuclease which has nicking activity comprising the steps of:
(a) identifying a heterodimeric Type IIT endonuclease;
(b) identifying a conserved region within said Type IIT endonuclease; wherein said conserved region is **characterized by** the sequence EXK;
(c) generating at least one mutation within said conserved region; and
(d) analyzing the mutant endonuclease of step (c) for nicking endonuclease activity.

2. A method according to claim 1 of making a mutated Type IIT endonuclease which has nicking activity;
wherein the endonuclease comprises two different protein subunits, wherein a first protein subunit comprises a catalytic domain capable of cleaving a first strand of a double stranded DNA, and wherein a second protein subunit comprises a catalytic domain ***incapable*** of cleaving a second strand of a double stranded DNA;
the method comprising the steps of:
(a) identifying a heterodimeric Type IIT endonuclease; wherein the endonuclease comprises two different protein subunits, wherein a first protein subunit comprises a catalytic domain capable of cleaving a first strand of a double stranded DNA, and wherein a second protein subunit comprises a catalytic domain ***capable*** of cleaving a second strand of a double stranded DNA;
(b) identifying a conserved region within said Type IIT endonuclease; wherein said conserved region is **characterized by** the sequence EXK;
(c) generating at least one mutation within said conserved region; and
(d) analyzing the mutant endonuclease of step (c) for nicking endonuclease activity.

## Patentansprüche

1. Verfahren zur Herstellung einer mutierten Typ IIT Endonuklease, die Nicking-Aktivität hat, umfassend die Schritte:
(a) Identifizieren einer heterodimeren Typ IIT Endonuklease;
(b) Identifizieren einer konservierten Region innerhalb der Typ IIT Endonuklease; wobei die konservierte Region durch die Sequenz EXK **gekennzeichnet** ist;
(c) Erzeugen zumindest einer Mutation innerhalb der konservierten Region; und
(d) Analysieren der mutanten Endonuklease von Schritt (c) auf Nicking-Endonuklease-Aktivität.

2. Verfahren gemäß Anspruch 1 zur Herstellung einer mutierten Typ IIT Endonuklease, die Nicking-Aktivität hat;
wobei die Endonuklease zwei verschiedene Protein-Untereinheiten umfasst, wobei eine erste Protein-Untereinheit eine katalytische Domäne umfasst, die einen ersten Strang einer Doppelstrang-DNA spalten kann, und wobei eine zweite Protein-Untereinheit eine katalytische Domäne umfasst, die nicht in der Lage ist, einen zweiten Strang einer Doppelstrang-DNA zu spalten;
das Verfahren umfassend die Schritte:
(a) Identifizieren einer heterodimeren Typ IIT Endonuklease; wobei die Endonuklease zwei verschiedene Protein-Untereinheiten umfasst, wobei eine erste Protein-Untereinheit eine katalytische Domäne umfasst, die einen ersten Strang einer Doppelstrang-DNA spalten kann, und wobei eine zweite Protein-Untereinheit eine katalytische Domäne umfasst, die in der Lage ist, einen zweiten Strang einer Doppelstrang-DNA zu spalten;
(b) Identifizieren einer konservierten Region in der Typ IIT Endonuklease; wobei die konservierte Region durch die Sequenz EXK **gekennzeichnet** ist;
(c) Erzeugen zumindest einer Mutation innerhalb der konservierten Region; und
(d) Analysieren der mutanten Endonuklease von Schritt (c) auf Nicking-Endonuklease-Aktivität.

## Revendications

1. Procédé pour produire une endonucléase de type IIT mutée qui a une activité de césure, comprenant les étapes consistant à :
(a) identifier une endonucléase de type IIT hétérodimère ;
(b) identifier une région conservée à l'intérieur de ladite endonucléase de type IIT ; ladite région conservée étant **caractérisée par** la séquence EXK ;
(c) générer au moins une mutation à l'intérieur de ladite région conservée ; et
(d) analyser l'endonucléase mutante de l'étape (c) pour une activité d'endonucléase de césure.

2. Procédé selon la revendication 1 pour produire une endonucléase de type IIT mutée qui a une activité de césure ;
dans lequel l'endonucléase comprend deux sous-unités protéiques différentes, une première sous-unité protéique comprenant un domaine catalytique capable de couper un premier brin d'un ADN double brin, et une deuxième sous-unité protéique comprenant un domaine catalytique incapable de couper un deuxième brin d'un ADN double brin ;
lequel procédé comprend les étapes consistant à :
(a) identifier une endonucléase de type IIT hétérodimère, laquelle endonucléase comprend deux sous-unités protéiques différentes, une première sous-unité protéique comprenant un domaine catalytique capable de couper un premier brin d'un ADN double brin, et une deuxième sous-unité protéique comprenant un domaine catalytique capable de couper un deuxième brin d'un ADN double brin ;
(b) identifier une région conservée à l'intérieur de ladite endonucléase de type IIT ; ladite région conservée étant **caractérisée par** la séquence EXK ;
(c) générer au moins une mutation à l'intérieur de ladite région conservée ; et
(d) analyser l'endonucléase mutante de l'étape (c) pour une activité d'endonucléase de césure.
